# EUROPEAN PATENT SPECIFICATION

(11) **EP 1 871 264 B1**
(45) Date of publication and mention of the grant of the patent: **21.09.2011**
(21) Application number: 06749690.1
(22) Date of filing: 11.04.2006
(51) Int. Cl.: A61B 18/22, A61N 5/06

(54) **OPTICAL PROBE FOR DELIVERY OF LIGHT**
OPTISCHE SONDE ZUR ABGABE VON LICHT
SONDE OPTIQUE D'APPORT DE LUMIERE

(30) Priority: 04.04.2006 US 397768; 21.04.2005 US 673689 P
(43) Date of publication of application: 02.01.2008
(73) Proprietor: Ondine International Ltd., St. Michael (BB)
(72) Inventor: ROSE, Andreas, San Marcos CA 92078 (US); HERR, Guenter, D-35630 Ehringshausen (DE); RIDGE, Joe, Friday Harbor, WA 98250 (US); JOHNSTON, Kyle, Sammamish, WA 98074 (US)
(74) Representative: Bawden, Peter Charles
(86) International application number: PCT/US2006/013380
(87) International publication number: WO 2006/115761

(56) References cited:
- WO-A-00/25684
- DE-A1- 10 009 004
- US-A- 4 826 431
- US-A- 5 514 126
- US-A- 6 129 721
- US-B1- 6 358 272

## Description

### FIELD OF THE INVENTION

The present invention relates to an optical probe tip employed for the delivery of light. More particularly, the present invention relates to a probe tip and uses of the probe and/or tip for delivery of light to surfaces of tissue and other portions of the human body or other organisms.

### BACKGROUND OF THE INVENTION

Light delivery is an important aspect of many different procedures. Light delivery is particularly important for medical applications, such as surgeries, therapies, examinations or the like. As such, industry, and particularly the medical devices industry, has developed various different probes and probe tips for aiding in light delivery. However, many current probes and probe tips suffer from drawbacks. For example, many current probes and tips can often provide undesirable nonhomogeneous light distribution. As another example, many current probes and/or tips experience undesirably high levels of light loss. As other examples, many current probes and/or tips exhibit undesirably low levels of strength and flexibility and may be undesirably expensive. Therefore, the present invention provides, a probe tip that overcomes one or more of the aforementioned drawbacks or overcomes other drawbacks as will become clear upon reading the detailed description. WO 00/25684 discloses a probe tip having, inter alia, a non-monolithic structure.

### SUMMARY OF INVENTION

According the present invention provides a probe tip for delivery of light during medical applications, as defined in claim 1. The probe can include a hand piece, and a probe tip of the invention. The probe tip is typically designed to be removably fastened to the hand piece. The hand piece can include a body portion and preferably include a light source integrated with the body portion. The probe tip can include a base or cap portion and typically includes an elongated member extending outwardly from the cap portion. The cap portion of the probe tip typically defines an opening for receiving a portion of the hand set or vice versa. The elongated member and possibly the entire probe tip can be formed of an optical plastic or can define a tunnel or conduit suitable for the delivery of light.

### BRIEF DESCRIPTION OF THE DRAWINGS

The features and inventive aspects of the present invention will become more apparent upon reading the following detailed description, claims, and drawings, of which the following is a brief description:

Fig. 1 is a sectional view of a portion of an exemplary probe containing a probe tip according to the present invention.

Fig. 2 is sectional view of a portion of another exemplary probe containing a probe tip according to the present invention.

Figs. 3A, 3B and 3C are respectively, a perspective, a cut-away perspective and a sectional view of an exemplary probe tip and probe according to the present invention.

Fig. 4A and 4B are perspective views of two more exemplary probes of the present invention.

Figs. 5A and 5B are side views of another exemplary probe containing a probe tip of the present invention.

Fig. 6 is a sectional view of a portion of yet another exemplary probe containing a probe tip according to the present invention.

Fig. 7 is a perspective cut away view of another exemplary probe containing a probe tip according to the present invention.

Fig. 8 is a graphical depiction of exemplary light output of a probe containing a probe tip according to the present invention.

Fig. 9 is a representation of an exemplary technique for removal of a probe tip.

### DESCRIPTION OF THE PREFERRED EMBODIMENT

The present invention is predicated upon the provision of a probe, a probe tip or both that can be used for the delivery of light. It is contemplated that the probe and probe tip may be employed for the delivery of light in a variety of applications, however, the probe and tip are particularly useful for medical applications. As examples, the present invention can be employed for application of photo dynamic disinfection (PDD), photo dynamic therapy (PDT), photo activated antifungal therapy, photo assisted tissue welding, photo assisted bone and hard tissue development, photo assisted melting or polymerization of therapeutic compounds, photo curing in light curing cement applications (e.g., UV dental glue), photocoagulation of tissue in ophthalmologic related applications, optical sensing of tissue properties, optical Sensing and monitoring of diagnostic processes, combinations thereof or the like for the mouth or other body areas of humans or other animals. The present invention has been found particularly useful for performing the above medical applications and particularly, photo dynamic disinfection therapy, within the oral cavity of human or other animals.

Generally, a probe includes a hand piece and a probe tip according to the present invention. The probe tip is preferably attachable and detachable relative to the hand piece. The tip can be affixed to the hand piece via retention features (e.g., interlocking features) designed into a portion or member (e.g., a cap) of the tip, the hand piece or both and the retention features may be reusable or may have attributes so that a tip may not be reused. The tip can be constructed so that when it is attached to the distal end of the hand piece, a portion or member of the tip (e.g. a gripping section) acts as an ergonomically correct gripping surface. The gripping surface can surround the hand piece in such a fashion to act as a barrier so the hand piece is not exposed to contamination and does not necessarily need to be sterilized in certain circumstances. The tip is typically constructed of a translucent or transparent material that conducts input light to an emission area where light is emitted in a specific or predetermined pattern or the light may be collected. The shape of the tip may include regions that are tapered to reduce the size of the tip, reshape its light distribution characteristics, provide flexibility or a combination thereof. The shape of the tip may also include a contour or bend for facilitating the ergonomics of positioning the tip into specific locations in the mouth or other bodily regions. The distal portion of the tip may have surface characteristics that aide with shaping the pattern of the emitted or collected light. The tip may also serve to relay light back into the hand piece so that the return light may be interpreted to provide diagnostic information about the target location or the therapeutic process or both. The distal portion of the tip may also be functionalized with agents consisting of specific materials, chemical compounds, cells or other biological agents. The functionalizing agents may either be activated by the therapeutic light or may work by an independent process. The functionalizing agents may be used to assist a therapeutic application, a diagnostic application or both.

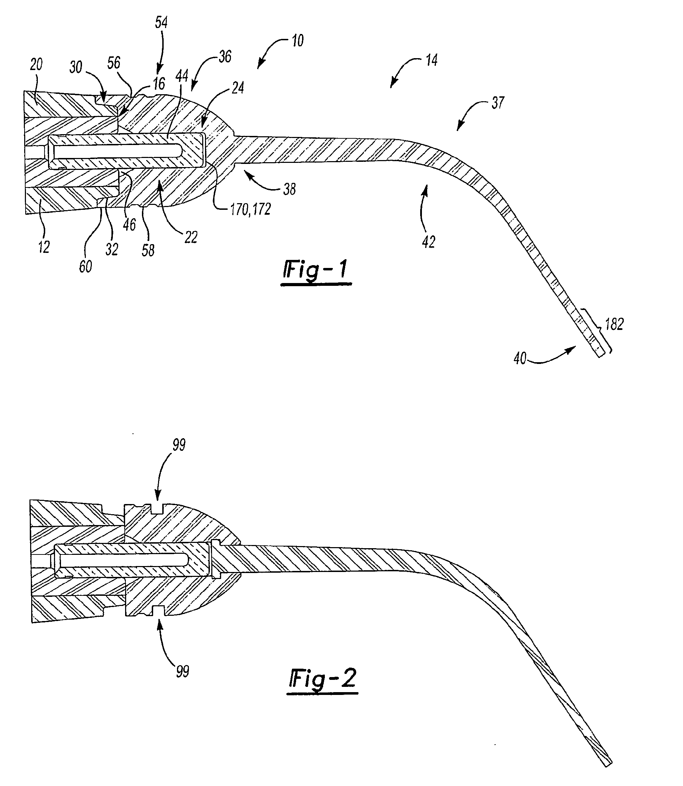

Fig. 1 illustrates one exemplary embodiment of a probe 10 containing a probe tip 14 according to the present invention. The probe 10 includes a hand piece 12 and a probe tip 14 removably attached to the hand piece 12. The tip 14 can be disposable or reusable as further discussed herein. As shown, the tip 14 is attached to the distal end 16 of the hand piece 12, however, it may be attached to another location of the hand piece 12. The hand piece 12 typically produces or relays light to the probe tip 14 such that the probe tip 14 can emit the light in a desired manner. Generally, the hand piece 12, the tip 14 or both can be employed to deliver light of any wavelength(s) including visible and invisible light, but is typically employed for delivering light having wavelengths between and/or including deep UV to Far IR.

The hand piece 12 typically includes a body portion 20 integrated with a light source 22 (e.g., a conduit for delivery of light to the tip). In the embodiment shown, the body portion 20 substantially surrounds the light source 22, which has a distal end 24 that extends outwardly from the distal end of the hand piece 12. The hand piece 12 is also shown to include an annular cavity 30 at least partially defined by a smaller diameter annular portion 32 at the distal end 16.

The tip 14 typically includes a base or cap portion 36 with an elongated member 37 extending outwardly from the cap portion 36 from a proximate end 38 to a distal end 40. The elongated member 37 is generally arcuate in shape and includes a bend 42. The elongated member 37 is also shown as being tapered along at least a portion or substantially the entirety of the member 37 such that the diameter of the member 37 becomes smaller via the taper traveling along the elongated member 37 from the proximate end 37 to the distal end 40.

Typically, the cap portion of a probe tip according to the present invention provides a structure that the user may grip for attaching the tip to the hand piece or removing the tip from the hand piece. The cap section may be flexible or semi-rigid, but is typically relatively rigid. The cap portion typically includes surface gripping features or contours. Such contours can include, but are not limited to, a knurled surface, a roughened surface or, as shown in Fig. 1, cavities and/or ridges There may also be contours (e.g., one or more cavities, edges or the like) suitable to aid in the removal of the tip by a mechanical tool. For example, the cap may be shaped with adjacent or adjoining flat surfaces in different planes (e.g., the cap may be shape as a nut, which may be hexagonal or have any number of planar surface arranged around the periphery of the cap). As another alternative, the cap can include one, two or more slots 99 as shown in Fig. 2 configured for receiving one or more portions of a tool for assisting in removing the cap, attaching the cap or both.

The cap section may be a portion of a monolithic single piece tip, as shown in Fig. 1. Alternately, the cap portion may comprise a second material (i.e. a silicon rubber gripping material) over molded onto or otherwise attached to the optical taper section or elongated member, resulting in an assembly similar to that shown in Fig. 2. The cap portion may also be a completely separate component that captures the optical taper and holds it affixed to the hand piece, also resulting in an assembly similar to that shown in Fig. 2.

A mechanical interface is typically employed for creating a physical mating or interference fit attaching the tip and the hand piece together. The mechanical interface may be a relatively tight tolerance radial symmetric structure (e.g. annular) similar to the cylinder pocket that mates with the standard fiber optic connector shown extending up to the input face in Fig. 1. Alternately, the mechanical interface may include different geometric cross sections (i.e. square pins) or non-symmetrical features that provide a solid interface while simultaneously creating a keyed alignment feature. These include, but are not limited to, double barrel configurations, prismatic barrels or the inclusions of various keyed geometries.

Retention features typically provide a method of at least temporarily maintaining or holding the tip onto the hand piece. There may be optional ribbing or other friction enhancing or interference fitting features that increase the friction between the tip and the handset. Fig. 1 shows a design where the tip is held in place simply by friction, vacuum pressure or a combination thereof, which can be created by pushing the tip and particularly the cap of the tip onto a cylindrical portion of the hand piece. Relatively tight mechanical tolerances can be created through molding of the tip, particularly the cap of the tip, such that, once a portion of the hand piece has been slid into a cavity of the tip (e.g., a cavity of the cap), vacuum pressure will resist any force that attempt to slide the tip off of the portion of the hand piece. For creating such vacuum pressure, it may be desirable for the portion of the hand piece to have a smooth outer surface. However, it is contemplated that mechanical feature or contours may additionally or alternatively be employed for holding the tip upon the hand piece. It is also possible to include features that inhibit the formation of vacuum pressure between the tip and the hand piece in situation where such pressure is undesirable.

In Fig. 1, the tip 14 is shown to define a first opening 44 in the cap portion 36 shown as a disc-shape open space, which is adjacent and continuous with a second opening 46 in the cap portion 36 shown as a cylindrical shaped open space. The cap portion 36 also includes a gripping region 54 that includes one or more openings 56 (e.g., annular cavities), one or more protrusions 58 (e.g., an annular protrusion or ridge) about the cap portion 36 of the tip 14. It is contemplated that adjacent and continuous with a third opening such as a tunnel could extend along and be substantially surrounded by the elongated member 37. It is typically preferred, however, that the probe tip be without any substantially opening down its length such that the outer surface or other portions of the elongated member 37 can act as a cladding for guiding light along the elongated member 37.

The tip 14 is fit upon the hand piece 12 such that the distal portion 16 of the hand piece 12 is received in the first opening 44 of the tip 14 while the distal portion 24 of the light source 22 is received in the second opening 46 of the tip 46. Preferably, the first opening 44 of the tip 14 is sized such that a portion 60 (shown as an annular portion) of the tip 14 is compression fit with or about the distal portion 16 of the hand piece 12 for removably attaching the tip 14 to the hand piece 12 as discussed above. In the particular embodiment of Fig. 1, the flange 60 friction fits over an annular portion 16 of the hand piece 12 for securely but releasably attaching the tip 14 to the hand piece 12.

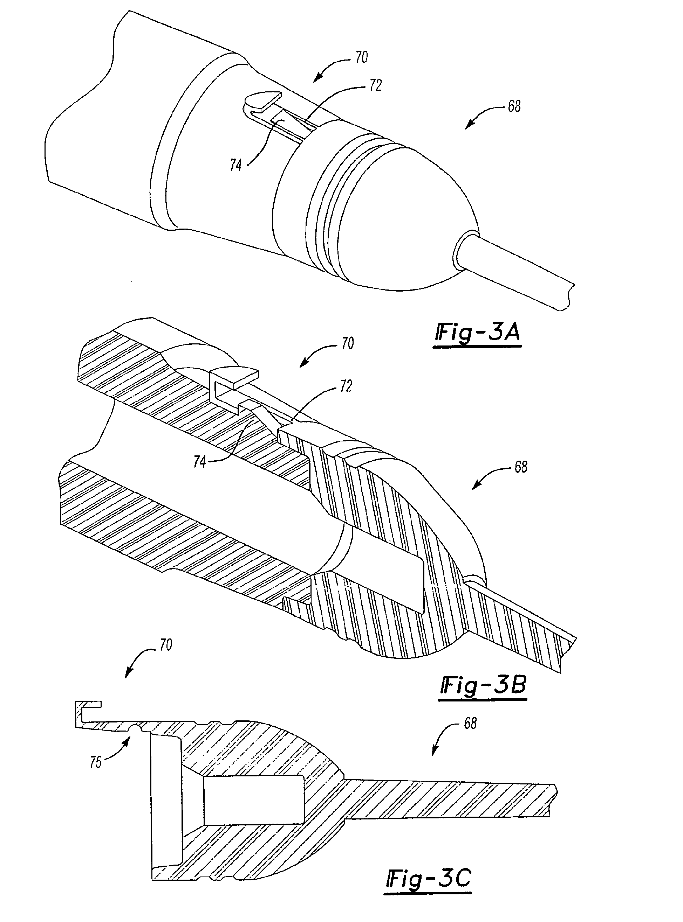

Additional or alternative features may be employed to aid with retention of the tip to the hand piece and may be designed and constructed in the tip, the hand piece or both. Without limitation, Figs. 3A-3C, Fig. 4, Fig. 5 and Fig. 6 show several other classes of retention features covered by this invention that go beyond the friction and vacuum techniques discussed previously. Figs. 3A-3C show views of a tip 68 designed with a tab 70 having a slot 72 in it that engages with a corresponding post feature 74 on the hand piece. When engaged, as in Fig. 3B, the tip is held firmly in place. When the tab 70 is lifted, the tip 68 is easy to disengage. Figs. 3A and 3B show a tab/post structure where the tab 70 is radially deflected during the engagement/disengagement process.

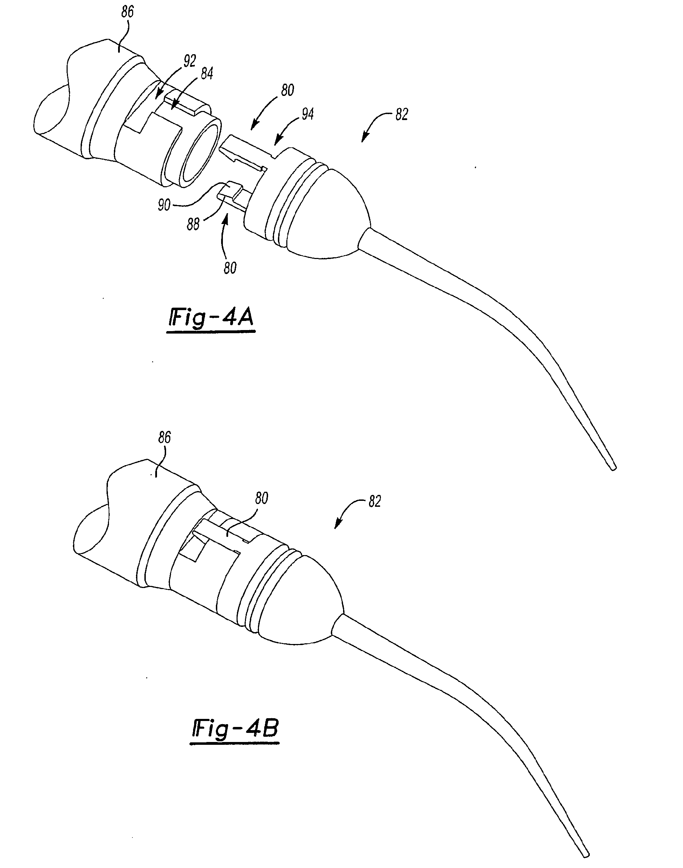

Fig. 4 also shows a retention configuration where a tab 80 on the tip 82 is radially deflected outward during engagement. In this configuration, there are two opposed tabs 80 on the tip 82 that are first aligned with the axial slots 84 in the hand piece 86. When the tip 82 is pushed onto the hand piece 86, ramps and/or chamfered surfaces 88 on teeth 90 extending from the inside surfaces of the tabs 80 cause the tabs 80 to deflect outwards. When the tip 82 is fully engaged, the teeth 90 clear the axial slot 84 and firmly seat in a deeper slot 92 that runs perpendicular to the axial slot 84 while the body portion of the tabs 80 is located in the axial slots 84. The tip 82 is now interference fit and/or relatively securely retained onto the hand piece 86.

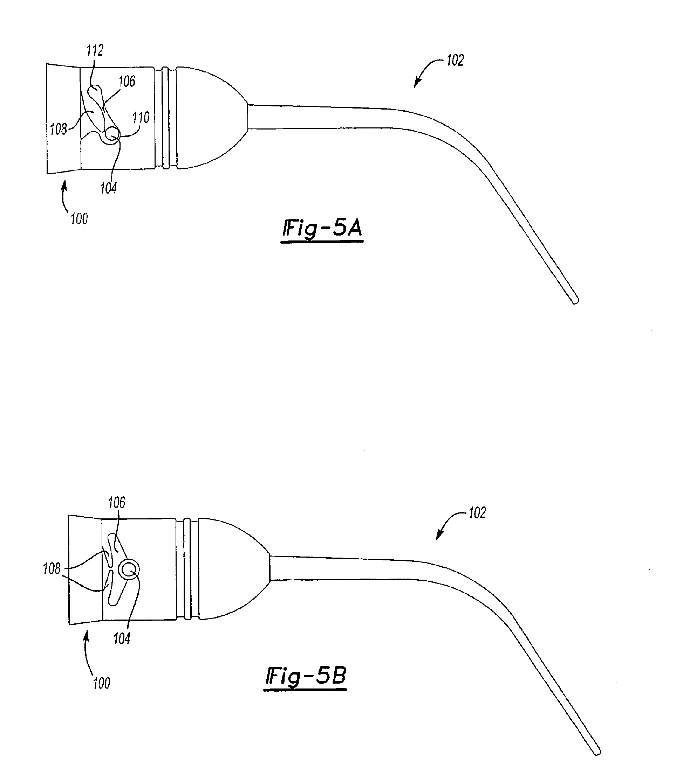

Fig. 5B shows an example of a linear valve style of engagement where features in the hand piece 100 engage features on the tip 102 when it is inserted into the tip. Fig. 5A shows an example of a linear type of engagement where the tip 102 is retained when the hand set is pushed into the tip. However, the design in Fig. 5A requires only a slight modification to form a rotary engagement mechanism where the tip 102 is retained by rotating the tip 102 so that a feature 104 (e.g. protusion) on the hand piece engages in a pocket 106 on the tip.

To assist in avoiding cross contamination, it is desirable that the option exist so that tips covered by this invention are used only once (e.g., used in a medical application for a single person or animal) and then disposed of. In order to enforce only a single usage of the tip, it is within the scope of this invention that features be engineered into a tip's design so that after the first use, it cannot be readily used again.

The device in Fig. 3A and 3B can be optionally made into a single use design by including appropriately designed features into the tip. Close examination of Fig. 3A will reveal a notch 75 in the tab 70. Without limitation, this is an example of a feature that will make the tab 70 act as a hinge. The tab 70 is strong enough to allow it to engage and provide retention for the first use, but when it is pried up to remove the tip 68, it will plastically deform, e.g. a stressed hinge will form in the vicinity of the notch 72. The retention tab 70 will now have lost the memory that keeps the tab 70 straight and provides the retention force to keep it engaged to the features on the hand piece. Therefore, there is a visual clue that the tip 68 has been used and, if the hand piece is re-inserted into the tip 68, engagement and/or retention of the tip 68 with the hand piece is inhibited such that the potential re-use of the tip 68 is also inhibited.

In a similar fashion, the tip 82 in Figs. 4A and 4B has weakened sections 94 designed into the base of the tabs 80 where they join the cap. When the tip 82 is either rotated or pulled axially, the tabs 80 will plastically deform (e.g. either break or form a stress hinge) at the weakened location 94, releasing the tip 82 from the hand piece. This tip design also provides a visual clue that the tip 82 has been used and the deformed tabs 80 will also inhibit reuse of the tip 82. This type of "push on, twist off" style of retention is very desirable for ease of use by the clinical technicians.

As another example, the tip retention mechanism shown in Fig. 5B can also be designed for single use. In this particular "linear valve" example, the retention feature on the hand piece is a type of post 104. The pair of flexible arms 108 shown as part of the tip 102 will flex inward when the post 104 is inserted. The amount of deflection is not enough to harm the arm material, so the arms 108 will retain a spring force that will help retain the post 104 in the pocket 106. However, when linear force is applied to the post 104 to remove it from the pocket 106, the arms 108 have to deflect significantly further than during insertion. This is enough deflection to overcome the strength of the material, causing the arms 108 to plastically deform. As examples of such plastic deformation, a stress hinge can form so the arms 108 loose their spring force but remain attached or one or more of the arms 108 will break clean off. This allows the tip to be removed and provides a visual clue the tip 102 has been used. If the hand piece is re-inserted into the tip, engagement and/or retention of the tip 102 with the hand piece is inhibited such that the potential re-use of the tip is also inhibited.

The device in Fig. 5A is a one sided variety of "linear valve" that works as a single use device in a similar manner to the design in Fig. 5B. Here, there is only a single arm 108 that deflects a safe amount when the post 104 is inserted, so it provides sufficient spring force to help retain the tip 102 or post 104 in the pocket (as shown). The arm 108 must deflect past the yield point of the material when the tip 102 is removed, causing the arm 108 to plastically deform. As examples of such deformation, a stress hinge can form so the arm looses its spring force but is retained or the arm 108 may break clean off. Either allows the tip to be removed and provides a visual clue the tip 102 has been used. If the hand piece is re-inserted into the tip 102, engagement and/or retention of the tip with the hand piece is inhibited such that the potential re-use of the tip 102 is also inhibited.

The device in Fig. 5A suggests features that can be designed into a tip to form a single use tip with a "rotary valve" style of retention figure. Examination of Fig. 5A shows that when the hand piece is inserted into the tip 102, the post pushes past a first face of the hinge feature 108, causing a small amount of deflection, and settles into a first position or location 110. This amount of deflection is not enough to damage the material in the hinge section 108 and the hinged feature retains a slight spring force. When the tip 102 is twisted to lock it in place, the post pushes past a second face in the hinge feature 108, again causing a relatively small amount of deflection. The post 104 is now securely captured in a pocket 106 in a second location 112 by the spring force of the hinge for holding the tip 102 onto the hand piece.

Several methods can be used to disengage the tip. As with all the designs shown, axial force may be applied to pull the tip off the hand piece, possibly with the aide features similar to the slots shown in Fig. 2. This removal force will force open the hinge section far enough to plastically deform one or more retention features, releasing the post and forming a stress hinge in the hinge material or retention feature. The result is that the hinge will stay "open" and it will be difficult for the tip to re-engage with the hand piece a second time. Alternately, the hinge can be designed to plastically deform by breaking when the axial force is applied, so the tip is disengaged and reengagement is inhibited. Another method of disengaging the tip is to rotate the post back to its first position, then pulling the tip off. In the design shown in Fig. 5A, the post will slip past the second face of the hinge without damaging it. However, the hinge is designed so that it must open further to let the post out than it did to let it in. This further amount of deflection is typically enough to cause plastic deformation such as a stress hinge, causing the hinge to loose its retention force after the tip is removed the first time. Thereafter, the tip will be inhibited from securely engaging with the hand piece a second time.

The device in Fig. 5A suggests features that can be designed into a tip to form a single use tip with a "push on, twist off" style of single use retention feature. Examination of Fig. 5A shows that when the hand piece is inserted into the tip, the post pushes past a first face of the hinge feature, causing a relatively small amount of deflection, and settles into a first position. The arm can be designed to clamp the post in the pocket, providing secure retention of the tip onto the hand piece, as previously discussed. To remove the tip, a rotary motion is used to force the post towards the second position. This motion might be aided by the use of a tool (e.g., a wrench) that engages features in the cap, such as the slots shown in Fig. 2 or a plural planar sided (e.g., nut) pattern (i.e. hexagonal pattern of facets) molded into the cap. The motion of the post towards the pocket will force the arm to deflect until it is stopped by the other features on the tip. As the twisting motion continues, the hinge portion of the arm will be under increasing stress. The arm geometry can be specifically designed with a weak point, so that when an appropriate amount of tension is applied, the hinge section will plastically deform (e.g., break), allowing the post out and the tip to disengage from the hand piece. This will provide a visual clue the tip has been used. If the hand piece is re-inserted into the tip, engagement and/or retention of the tip with the hand piece is inhibited such that the potential re-use of the tip is also inhibited.

Note that all the proceeding examples can be designed to work with either a single engagement feature or with multiple engagement features. In each case, the properties of the construction material combined with the geometrical design of the pocket(s), arm(s) and post(s) will determine if the device is suitable for multiple uses or only a single use. The specific design needed to create a single use feature is variable with the amount of deflection needed to pass the post in either direction. Specifically designed weakness in the hinge section or other location in the arm will typically determine if the arm will plastically deform and if so, with what force and if it will break away, bend or the like. The characteristics of the weakened arm can be variable with the strength by material, dimension of the beam and dimensions of the joint or dimensions of specific weakening features such as a notch.

With the aid of the present disclosure, it is contemplated that someone practiced in the art of designing molded parts will be able to imagine other single use or multiple use retention features within the scope of the present invention. It should be noted that experimentation with different materials can dictate the parameters of a specific design. For example, it has been found that when given a molded polymethyl methacrylate (PMMA) beam that is 1 mm x 0.75 mm with a 0.25 mm deep triangular notch in the 1 mm dimension, the beam can deflect 15° without significant or substantial plastic deformation. However, at approximately 20° deflection, the beam will typically plastically deform at the location of the notch, forming a stress hinge. In another example, it has been found that a PMMA beam with dimensions of 1.5 mm x 1.5 mm and a 0.5 mm notch was found to plastically deform somewhere above 10° of deflection. It was found that if the notch was deeper than 0.5 mm, a clean break would occur, e.g. the beam would be severed. While PMMA is one preferred material, almost any other transparent or translucent material or a combination thereof, may be used including but not limited to other plastics, moldable polymers, epoxies, curable urethanes, etc.

It should be understood that the term plastic deformation, as used herein, is meant to describe a deformation of a material wherein the material does not substantially return to its original shape or configuration and the term can be used in reference to any material including plastics, metals or other material unless otherwise specifically stated.

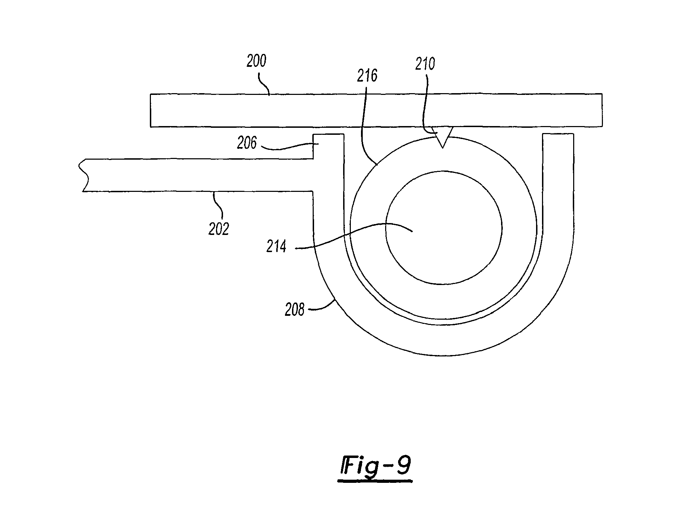

It should also be noted that with any of the tip designs covered by this invention, the tips can be removed from the hand piece by a device which cuts them off. Without limitation, one method of accomplishing this is to utilize a device configured for contacting at least one blade section with the tip. On such device could include a pair of jaws and, optionally a handle connected to each of the jaws. When the tip/hand piece assembly is placed into the jaws, a first jaw could be shaped to cradle the tip. When the jaws are moved toward each other, (e.g., using the handles), the blade in a second jaw would be driven into the tip material in such a fashion as to cut the tip. The blade would preferably be of a length or be otherwise configured so that it would be too short to contact the material of the hand piece when the jaws are moved toward each other. In this fashion, by putting the tip into jaws and closing them, the tip can be cut and removed from the hand piece, without damaging the hand piece. The tip would thereafter typically be unsuitable for subsequent reuse. An exemplary depiction of this embodiment is shown in Fig. 9 to include an upper jaw 200, a lower jaw 202, a mechanical stop 206, a cradle 208, a blade 210, a hand piece 214 and a tip 216.

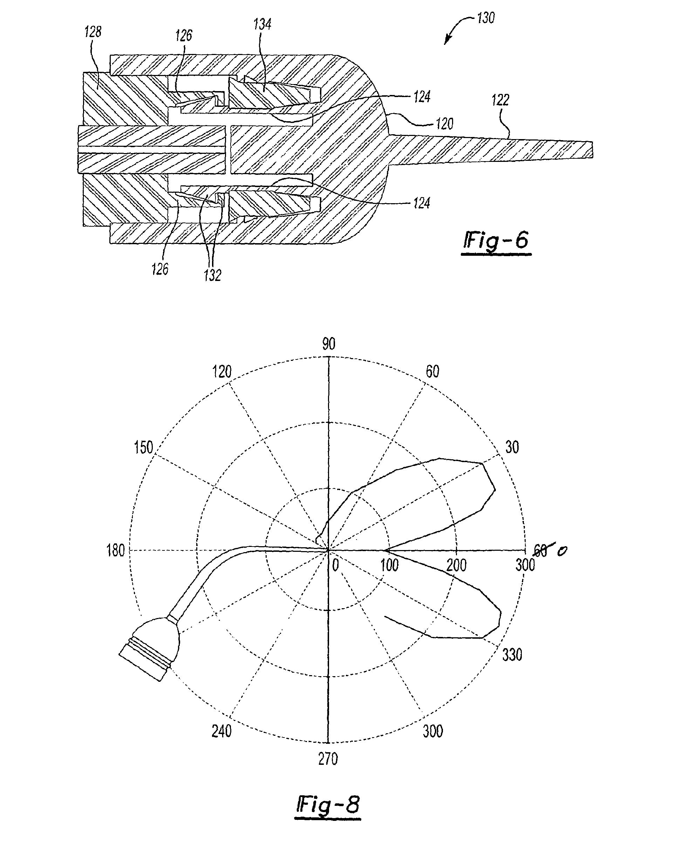

Another class of tip designs, an example of which is shown in Fig. 6, would also result in a single use or disposable tip design. Fig. 6 deals with the cap 120 and elongated member shown as a taper 122 as a single unit, regardless of their construction. The tip has deformable interlock arms 124 that engage in a hand piece catch feature 126 when the hand piece 128 is inserted into the tip 130. Without limitation, the example in Fig. 6 shows the arms 124 in the tip 130 engaging inside the features or extensions 126 on the hand piece 128. In the embodiment, shown, the interlock arms 124 and the hand piece catch 126 are cantilevered extensions with interlock flanges 132 at their distal ends.

A second piece 134 is also included in the tip. This piece can be of any appropriate material and, without limitation, is depicted as a cut away of a release ring structure 134 in Fig. 6. As shown, this release ring 134 has a shape such that when it is pushed over the deformable interlock arms 124, it is retained by the tip 130, making an assembly suitable for commercial distribution. When the release ring 134 is driven farther into the tip 130, the release ring 134 will engage with the deformable interlock arms 124 in such a fashion as to force the deformable arms 124 into a state where they no longer engage with the hand piece catches 126. As shown, the release ring 134 has further features so that when it is driven far enough into the tip 130, it will engage with the tip catch or cavity 138. In this fashion, the release ring 134 is captured in the tip 130 and the deformable interlock arms 124 are biased inwardly in a state where they will not be suitable to re-engage with the hand piece catches.

In Fig. 6, there is enough room provided so that by providing axial force to push the tip 130 firmly onto the hand piece 128, the ends of the hand piece catches 126 push against the release ring 134 to engage it into the tip catch features 138. In this fashion, the tip 130 is pressed firmly onto the hand piece 128 to disengage it and allow its removal. If insufficient axial force is provided, the tip 130 would not typically disengage and the technician would have to press harder. Preferably, only when the tip release ring 134 was held within the internal cavity of the tip 130 would the tip 130 be released from the hand piece. In this fashion, the tip 130 would not be retained onto the hand piece 128 a second time, and would thereafter not be suitable for subsequent reuse.

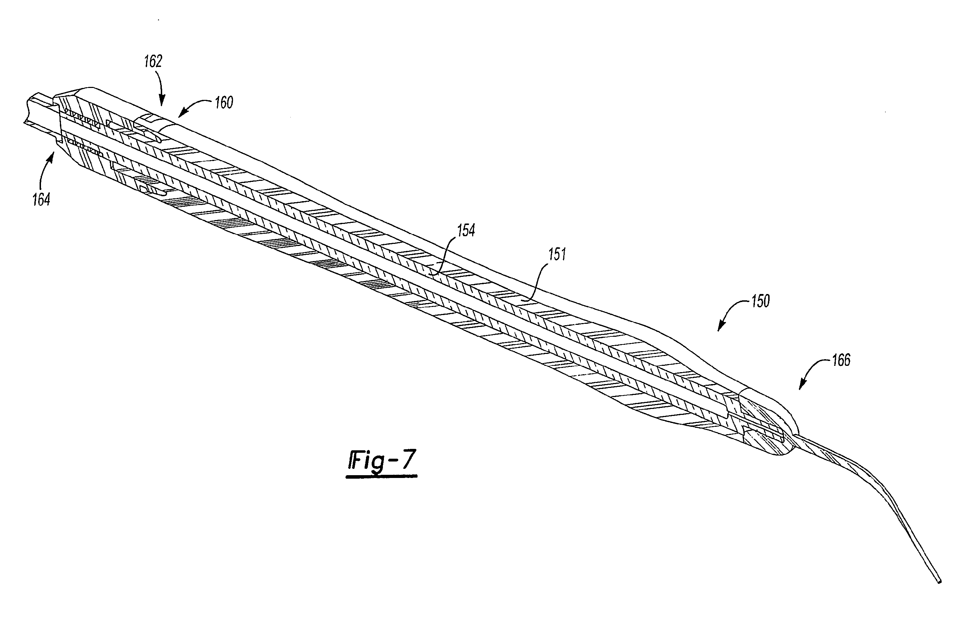

Fig. 7 shows a version of a tip 150 that is similar to that shown in Fig. 1 except the gripping section 151 of the cap 152 has been elongated to cover a significant portion of the hand piece 154. The mechanical interface between the hand piece 154 and the tip 150 extends substantially or almost the entire length of the hand piece 154. The same single use retention features 160 as shown in Fig. 4 are used, with the interface 162 being located near the proximal end 164 of the hand piece 154. Note that any of the disclosed retention strategies disclosed above could also be effectively utilized.

It is within the scope of this invention that the shape of the gripping surface can be varied in texture, surface finish, surface relief patterns and surface contours to promote a firm grip while providing an ergonomically comfortable grip. Without limitation, radial grooves, knurling patterns or roughened surfaces may be employed. The tip may be constructed as a single piece, e.g. a single molded part, or the tip may be constructed from several materials or in several pieces. Without limitation, the gripping section 151 may be or include an elastomer (e.g., a silicone rubber) or other compliant material while the rest 166 of the tip 150 is typically formed from an optical plastic such as those discussed herein. Alternatively, the entire tip structure 150 can be formed as a single piece and a compliant gripping section 151 can be created by a secondary processing step such as an over-molding process. The tip can also be constructed of several pieces that are affixed together. Without limitation, the gripping section could be a clam shell that is glued together, or the shells could have interlock features that hold them together at one end and be held together by the cap section at the other.

It is within the scope of this invention that the length of the hand piece covered by the tip can be varied as a design option. However, if enough (e.g. 40%, 60%, 80% or greater) of the hand piece is covered by a continuous, substantially impervious tip, the tip will act as a barrier, protecting the hand piece from biological contamination. This can provide a significant benefit of lower manufacturing prices for the hand set because it does not need to be designed to withstand repeated trips through an autoclave for sterilization. The disposable tip and the removal of the need for sterilization can also save significant technician time.

The input face, an example of which is shown at numeral 170 in Fig. 1, is the optical interface between the taper section 37 and the gap 172. As shown, the surface of the input face 170 may be a flat, smooth surface, but may also be contoured, if desired. Alternatively, it is within the scope of this invention that the surface may have various light redirecting features constructed into it or applied to it. Without limitation, these may include one or a plurality of convex lens structures that helps gather and direct light passing though the Input Face. The interface may also include one or a plurality of concave structures that convert the light passing though the Input Face into higher propagating angles. i.e. to aide with generating the optimal light distribution pattern from the emission area. The interface may have a deliberately rough surface constructed upon it or applied to it in order to cause scattering that would modify the propagating angles of the light to generate a more optimal light distribution pattern from the emission area. The features on the interface may be random in nature or consist of a either a single or a plurality of prismatic or lenticular facets. Further, the features on the input face may form a Fresnel lens or a holographic element.

All of these features on the input face may be formed directly into the material of the taper or may be applied as a separate layer or component. Further, it is also within the scope of this invention that the surface may also be coated to adjust the reflection, refraction, diffraction scattering, transmission or absorption properties of the input face. These coating may include anti-reflection coatings, the properties of which are well know in the art. The coatings may also include spectrally active coatings such as those that act as wavelengths filters and are made from either layered assemblies of transparent materials or layers of dyed molecules.

The gap is the region between the source (the optical interface(s) on the hand piece) and the Input Face. The gap may be very small, e.g. allowing contact between the Light Source and the Input Face, or even non-existent. Alternatively, the gap may also be fairly large. Where a large input aperture is employed, the larger gap can be used and can minimize throughput losses or decreases in the quality of the light pattern at the emission area.

It is also within the scope of this invention that a polymer, gel or liquid may be used to at least partially fill that gap in order to adjust the coupling/back reflection properties. Advantageously, such an embodiment can bring the refractive indices of adjacent optical media closer and potentially lower the reflection losses when light crosses the interfaces of the media. Also, an index matching cement may be used to additionally or alternatively improve coupling performance and potentially assist in locking the tip to the handset.

The taper section or elongated member is the structure that guides the light from the input face to the emission area. Although the cap is not required to conduct light and therefore may be of transparent, translucent or opaque materials, the taper is preferably designed to conduct light toward the emission area without undue losses. Therefore, the taper is typically constructed of relatively transparent or highly translucent materials. Examples are, without limitation, acrylic or acrylates (e.g. PMMA), styrenics (e.g., polystyrene) polycarbonates, poly (vinyl chloride), epoxies, urethanes, Sol Gels, etc., combinations thereof or the like. It is within the scope of this invention that, in part through choice of materials, the Taper can conduct any combination of optical wavelengths, e.g. short UV (< 0.2 um) to far IR (> 10um). It is also with the scope of this invention that the taper conduct light from the input face to the emission area or from the emission area to the input face, or both. Furthermore, it is contemplated that a cladding may surround the taper for assisting in guiding light.

It is contemplated within the scope of this invention that the taper or elongated member may have any combination of longitudinal and cross sectional shapes. Without limitation, it may have sections that are straight walled, have a continuous taper, have shallow draft (for pulling from the mold), have various geometric features (i.e. raised or depressed sections) or have a mixture of different geometries. Without limitation, the cross section of the Taper may have any combination of circular, oval, elliptical, various polygonal and/or prismatic shapes. The Taper may also have a combination of different cross section shapes along its length and may have a continuously varying shapes such as a slow, longitudinal twist. One reason for using different cross sectional shapes is to help mix the propagating light to homogenize the spatial and angular content of the output pattern. Square sections intermixed with circular sections can be especially effective at this kind of mixing, but a similar benefit can be gained from other combinations of dissimilar cross sections.

It is within the scope of this invention that the length and cross section sizes of the taper or elongated member are limited only by needs of the application and the requirement of maintaining physical integrity. As a non limiting illustrative example, an unsupported 500 um PMMA taper that is 1 inch long would probably deliver the appropriate illumination but would be too flimsy for a technician to effectively guide into periodontal pockets. However, such a taper geometry may be suitable for applying illumination to an optical cement curing application. It should be noted that both the cross section diameter (or width) and the length of the taper will affect the flexibility of the taper. A taper that starts with a relatively larger diameter and ends up with a relatively smaller diameter will typically be most flexible and hence undergo more deflection in the vicinity of the tip, whereas a long section of a relatively large diameter or consistent diameter taper will typically undergo similar deflection along its entire length.

It is preferred, although not required, that the light propagating though the taper be contained by total or substantially total internal reflection (e.g., greater than 80, 90 or 95 percent internal reflection). One way to assist in accomplishing such internal reflection is by having, typically through material choices, the refractive index differential between the taper (the core) and the surrounding environment (the cladding) be relatively high. An illustrative example, with out limiting the scope of materials used in this invention, is a PMMA to AIR interface with a refractive index range of 1.49 to 1.0. Using Snell's law, it can be shown that PMMA in AIR will propagate angles of almost 58° relative to the axis of the Taper. Even if the entire length of the Taper is immersed in water based fluids, the PMMA to WATER interface will still allow propagation of light up to 26°. Therefore, an unclad, single material Taper may be employed to efficiently propagate and deliver light of a numerical aperture of at least 0.45 NA.

A bend section is a single or plurality of regions where the Taper is bent. The bend can improve ergonomics and allow reaching difficult areas. The Bend may be omitted or there may be multiple or compound Bends. In theory, there is a maximum angle for a Bend section where light still guides inside the Taper section. This angle is related to the maximum angle for total internal reflection. Practically, the limit to the Taper is actually a function of the radius of curvature as well as the angle of deviation. As an illustrative example, the bend seen in Fig. 1, is approximately 60°, yet due to the 20 mm diameter of curvature, no excessive propagation loss is detected when the device is constructed of PMMA. If the device had been constructed with an abrupt 60° bend, light could spill out at the joint section. However, since it is in the scope of this invention to include over coating constructed as mirrors, such an over coat could be used to keep light in the Taper, even if it were formed with a "Z" profile.

An emission area is the region where the taper emits light propagating in the taper or collects light into the taper or both. The taper can be designed with specific regions where light is intended to be emitted. This region could be the distal end, a region near the end or at any place along the taper or a combination of locations. The emission area may cover the entire circumference of a section of the taper tip or may occur in limited regions. These regions may extend along portions of the length of the taper or along its entire length. A plurality of emission areas may exist.

The emission area, shown as 182 in Fig. 1, may be caused to emit light by an area where the surface of the taper is specifically modified so that light spills out, i.e. at a discontinuity. Without limiting the scope of this invention, these features may include a roughened surface (i.e. a random 32 um "sand paper" surface roughening), patterns of bumps, patterns of pits, patterns of slots running longitudinally or radially, helical "threads", longitudinal corrugations, prismatic features, lenticular features, or even surface relief features resembling Fresnel lenses or holographic elements. The portion of the emission are that is roughened is typically between about 3 mm and about 12 mm (e.g., about 7 mm), although not required. Moreover, the tip may be roughened on an external surface or internally for elongated members with internal openings.

The emission area(s) may preferably be formed by features in a mold, i.e. a specific region of the mold where the normally highly polished surface that forms the taper gives way to a section with 32 um surface finish. These features may be reproduced in the molded taper, providing the emission area. Alternatively, other techniques may be used to create the emission area, including but not limited to chemical treatments (such as etching), laser treatment (such as writing a pattern), mechanical treatments (such as mechanically roughening an area with an abrasive), or electronic discharge (such as an ionic discharge). The treatments may be to the mold or to the individual tapers. The treatment may be done to the native material of the taper or to a material that is generally or selectively applied to the surface of the taper, such as a polymer film or ceramic film. These films may be the same films used to overcoat the taper or may be a separate material. The emission area may also be formed by combination of procedures. This is exemplified, without limitation, by forming a rough surface using features in the mold, then smoothing them slightly using a post process heating treatment.

Alternatively, it is also possible to form the emission area by modifying the material the taper is made of. This could be accomplished in the device shown in Fig. 2 by molding the Emission Area from a material with a high scattering coefficient and molding the rest of the Taper from second, preferable transparent material. Examples of appropriate scattering materials are, with out limitation, translucent materials with inherent light scattering elements or characteristics or transparent materials that typically includes one or a plurality of light scattering elements dispersed within the material. Examples of light scattering elements include, without limitation, aluminum compounds, oxides (e.g., titanium oxide, aluminum oxide, barium oxide), ceramic, polymers, masses (e.g., beads, balls or spheres) of higher or lower refractive index than the fill material (e.g., sapphire balls, hollow microspheres), combinations thereof or the like.

Alternatively, it is also possible to cause the taper to emit light by forcing the propagating light to exceed the allowed numerical aperture of the taper. This can be arranged by putting specific tight radius bends into the taper. This can also be arranged by rapidly increasing the rate at which the cross sectional diameter of the taper decreases.

It is within the scope of this invention that the pattern of light from the emission area may be a spatially uniform pattern with a high degree of angular uniformity. It is also within the scope of this invention that the light may emit from the emission area at one or a plurality of locations. Further, the emitted light may have diffuse angular distributions or narrow angular distributions or combinations thereof. The tip may be used to create one or a plurality of specific illumination patterns. It may be used to disperse light or to concentrate it or both.

It is within the scope of this invention that all the features in the emission area can be use for dispersing light out of the taper or for collecting light into the taper or both. Any combination of the features mentioned with respect to this invention may be design to aid with the collection of light or the emission of light. These include features and techniques not specifically mentioned but which are extension of disclosed ideas that would be known to one practiced in the art of optics.

When emitted, it is typically preferable that the light be emitted in a relatively uniform distribution over a region to avoid excessive light exposure in one area or unsatisfactory light exposure in an area. Advantageously, some of the features above can assist in creating such a distribution. The probe can also include a control feature for limiting the amount of light to, through or emitted by the probe. One or more of the features discussed above or elsewhere herein can assist the probe in exhibiting relatively low loss of light. In a preferred embodiment, the design of the probe tip can assist in ensuring that a substantial portion (i.e., greater than 70%) or substantially all (i.e., greater than 95%) of the light launched into the probe tip makes it to the distal end of the probe tip wherein and it may be scattered out through the roughened section of the tip.

In one embodiment, the conical taper or elongate member forms what is a substantially an air clad light guide with a relatively high numerical aperture (NA). As such, if typical bodily fluids or otherwise are in contact with the taper surface, the taper can still act as a cladding and the device can still guides most or substantially all of its light to the distal end of the tip.

The distal end of the tip may have features designed to scatter, redirect, absorb or internally reflect the light. The features may be lenticular or prismatic and may include an increase in the cross section of the taper (i.e. a ball end larger than the size of the taper near the tip). The features may be designed to disperse light or to concentrate it or both. The distal end may emit light or collect light or both. The distal end may be over coated with a material or pattern that modifies the transmission, absorption, reflection, diffraction or scattering of light that is emitted or collect. Without limitation, an example would be a distal end formed as a small radius with a metalized mirror over coating. This would convert forward propagating light into a higher NA propagating back towards the input face. This type of design would help increase the angular dispersion of light emitted from the emission area.

Functional Coating: It is within the scope of the present invention that either the emission area or the distal end or both can be treated with a function coating to extend the utility of the tip. Without limitation, these coatings may that aid with therapy or be used in sensing or both.

Without limiting the scope of this invention, an illustrative example of a functional coating that could be applied to a tip to aid in therapy would be a thin Sol Gel film applied to the emission area, where the film is used to entrap photo sensitizer molecules to assist with a PDD application.

Sol Gel films are ceramic films that can be engineered to have very specific properties, including very specific porosity characteristics. The Sol Gel material starts as a solution (e.g. a slurry) that can be "doped" with useful dye molecules, i.e. methylene blue (MB). The solution can be applied to a tip by various methods including dip coating. By varying parameters such as the viscosity of the solution, a repeatable coatings with specific thicknesses can be formed that, when dry, will contain the dye molecules trapped in its matrix. When therapeutic light is applied through the tip (i.e approx. 660 +/- 40 nm light for MB) it activates the dye (i.e. MB will absorbed the light and creating singlet oxygen molecules). Depending on the porosity characteristics of the thin film, the active products of the dye are free to migrate into the surrounding tissue to assist in the sterilization process, even though the dye molecule remains trapped.

For such an application, there may be an advantage of engineering a relatively thin film so that the active products of the dye do not recombine inside the film. Also, utilizing a thin functional film or a low dye concentration or both will also allow a significant portion of the therapeutic light to radiate into the surrounding tissue to simultaneously assist in "standard" PDD with dye molecules that are not trapped by the thin film matrix. Additionally, the characteristics of the thin film can be tailored to assist with the scattering properties in the Emission Area.

Diagnostic Sensing: It is within the scope of this invention that the tip conducts light from the Input Face to the Emission Area or the Distal End or both, where it is dispersed it in an appropriate fashion. It is also within the scope of this invention that the tip can serve to collect light and conduct it back to the Input Face. This "return" light may be from any combination of light from the environment surrounding the tip, the Emission Area, the Distal End or from a Functional Coating. This collected light may be further relayed out of the tip and into the hand piece where it may ultimately be analyzed.

Without limiting the scope of this invention, the application of PDT presents an illustrative example of how return light collected by the tip may be useful. In some PDT applications, a dye such as Indocyanine Green (ICG) needs to be present in sufficient concentration in the vicinity of target tissue at the time of therapy in order to achieve effective tissue necrosis. In addition, PDT is less effective after a dye such as ICG has undergone a temporary or permanent bleaching process. Therefore, it is desirable to monitor the environment of the tip to determine if viable concentrations of active ICG are present.

One possibility for detecting if ICG is present is to analyze the return light collected by the tip. ICG has a peak absorbance at approximately 805 nm but it has a fluorescent emission spectrum with a peak at approximately 832 nm. It is possible to "pump" the ICG with optical radiation in a band that includes wavelengths lower than the fluorescent peak, i.e. below 820 nm. Simultaneously, the fluorescent emissions of the ICG can be analyzed by measuring amount of return light collected by the tip in a band higher than the pump peak, i.e. from 820 nm to 840 nm. When there is no ICG present, or the ICG that is present gets bleached, there is little or no fluorescence emission in the measurement band. Therefore, analysis of the light collected by the tip enables a diagnostic to verify if the therapy conditions are suitable for successful treatment.

The preceding examples should not in any way be taken to limit the scope of the types or utility of the functional films or the diagnostic sensing that can be accomplished with the present tip invention. For example, other parameters can be measured to gain diagnostic information about the environment around the tip besides measuring fluorescence. These include, without limitation, measuring either the spectral or the temporal characteristics or both for the fluorescence, auto-fluorescence, phosphorescence, emission, absorbance or scattering characteristics of the environment surrounding the tip.

Scope: The present tip invention can be used solely for therapeutic purposes or solely for diagnostic purposes or for therapeutic and diagnostic purposes as well as other purposes. The diagnostic light collection may provide information about the surrounding environment or the status of the Emission Area or Distal End, including monitoring functional films applied to the Emission Area or to the Distal End. The therapeutic and diagnostic processes can be dissimilar in nature, such as PDD can occur at one wavelength while monitoring a reflective dye Ph indicator is probed at a second wavelength.

The tip may be used for multiple therapeutic and diagnostic applications simultaneously. This may be accomplished by working with multiple emission wavelengths, multiple collection wavelengths or both. Various functional therapeutic or sensing films may be applied to the tip in spatially separate areas, i.e. without limitation, as stripes, dots or as different treatments to the emission area and the distal end. The various functional films may be mixed to produce a compound film with multiple abilities. Additionally, the emission and collection of light may be temporally varied to provide a form of temporal multiplexing that may be used instead of or in combination with spectral multiplexing.

The scope of this invention is not limited by the specific applications, materials, geometries, functional films or dyes mentioned above. It should be appreciated that one skilled in the art would be able to specify applications and configurations not specifically disclosed here but clearly within the scope of the invention. An example of such configuration would be a tip geometry designed to have the effect of concentrating light into a specific region for the purpose of tissue ablation.

### Example

Many aspects of this invention have been reduced to practice. One specific example that is well represented by the depiction in Fig. 1 is intended for a periodontal PDD application, although it may have other applications. In this case, the Hand Piece is terminated with a standard fiber optic connector with a highly polished OD2.49 mm cylindrical metal ferrule with a length of 5.43 mm.

The tip is a monolithic piece of PMMA formed by injection molding. The Cap engages the Hand Piece through friction and vacuum as shown, resulting in a Gap of 0.25 mm. The Input Face is a planer facet as large as the OD of a standard fiber optic connector. It is created with a very smooth surface during the molding process. The Taper has a 2.0 mm circular cross section. The section before the bend is approximately 11 mm long and the taper is approximately 1.5 mm at the start of the Bend. The Bend subtends a 60° angle with a diameter of approximately 20 mm. At the end of the Bend the Taper has a dimension of approximately 1.0 mm. The distance from the bend to the distal end is 17 mm. The Emission Area covers the last 7 mm of the Taper. It is finished with a 32 um surface finish created on the surface of the mold and transferred in the molding process. At the Distal End, the cross section is □0.6 mm with a spherical shape of the same dimension.

When illuminated with therapeutic light in a band around 660 nm, the far field emission pattern is as shown in Fig. 8. The tip demonstrates no spatial or angular hot spots in its emission pattern. It does not suffer from a dangerous hot spot axial to the Distal End. The output pattern of the tip reflects a design that was specifically optimized to emit most of its light down into the periodontal pocket where treatment is required. It has also been specifically designed with Distal End dimensions that are optimum for fitting into periodontal pockets. The Bend radius is optimum for reaching all location in the mouth. The Taper is specifically designed to make the Taper rigid before the Bend and flexible after the Bend. This also aids in reaching into the periodontal pockets while minimizing the chances of injuring the patients oral tissues.

It should be understood that each of the specific numbers given for the tip parameters in this specific example can be varied by large or small amounts depending upon the particular parameter. As a general guideline, it is contemplated that each of the parameters may be varied by ± 10 %, ± 20 %, ± 50 % or more relative to the value supplied in the example.

Depending upon the configuration, the probe may exhibit multiple advantages. The tapered shape of the elongated member can provide a relatively strong base with a relatively large optical input face. The end of the tip can be designed small enough to fit into tight spaces (e.g., periodontal pockets present in diseased gum tissue). The combined benefits of the input face, taper, distal end, emission treatment or combinations thereof can provide a desirable output pattern that can be customized to fit the need of a specific application, resulting in greater uniformity of the output light at the emission area and the distal end. Due to the inherent mixing in the taper section and the efficiency of the scattering area, the tip can be designed to assist in emitting a more homogeneous output even in the presence of a light source with relatively poor spatial or angular uniformity. Moreover, due to the use of a PMMA core with an air cladding, the allowed NA is typically between 0.5 and 1.0 (e.g., around 0.74), indicating an angle of between about 35 ° and 60 ° (e.g., almost 48°). This makes it possible to efficiently deliver light from a wide range or sources without the need for special source shaping optics.

The tapered shape can provide strength to the tip such that the tip can be substantially or entirely self supporting without the need for extra support elements (e.g., a needle sheath used in the prior art). Of course, such elements may be used, unless otherwise specifically recited. PMMA, when used and when combined with the size of the taper section, can produce a flexible tip. This can assist the technician in more accurately probing into sections of the gum or other tissue thereby increasing patient comfort by avoiding potential scrapes and poking. The design can be formed as a single piece molded in plastic, which can be relatively inexpensive. The molded plastic design, when used, lends itself to high volume mass production schemes, keeping costs down. Moreover, the molding process can typically be accurately repeated for producing tips that are consistent in size, shape or the like.

It is also contemplate that the tips can be formed at a sufficiently low price for allowing the tips to be disposable after use thereby avoiding the need for repeated sterilization and for assisting in avoiding cross-contamination. The gripping section can be configured so it surrounds and protects a portion of the hand piece. This can allows the hand piece to be safely reused without requiring repeated sterilization. This allows lower cost hand pieces and saves valuable technician time.

The materials in the probe are bio-compatible and have very little chance to produce an adverse reaction in the patient. The design of the tip, i.e. the taper and the bend, can allow an individual to comfortably reach and probe into the many crevices in the mouth or other body parts while minimizing patient discomfort. The design of the gripping section can allow the entire probe to fit the technicians hand comfortably and maintain a good grip during treatment.

The probe can allow for diagnostic optical sensing applications as well as monitoring of therapeutic processes when desired. The ability of the tip to pick up light from the application area and direct it back into the hand piece, combined with the functionality of the surface treatments to the emission area can allow the tip to be used in applications where diagnostics about a process or the status of the surrounding environment are beneficial.

It is also contemplated that additional or alternative features may be added or included. The probe tip may be configured for drug delivery through tip or through features back from the tip. The probe tip could be configured as a two part tip, for example, the optical tip could be overmolded with a cap section of other (nonoptical) material or the optical taper section could be held down by cap section "nut". The distal tip could be a mirror section to drive light emission back up towards the hand piece, thereby adjusting the output pattern.

Unless stated otherwise, dimensions and geometries of the various structures depicted herein are not intended to be restrictive of the invention, and other dimensions or geometries are possible. Plural structural components can be provided by a single integrated structure. Alternatively, a single integrated structure might be divided into separate plural components. In addition, while a feature of the present invention may have been described in the context of only one of the illustrated embodiments, such feature may be combined with one or more other features of other embodiments, for any given application. It will also be appreciated from the above that the fabrication of the unique structures herein and the operation thereof also constitute methods in accordance with the present invention.

## Claims

1. A probe tip for use with a hand piece that is connectable to a light source, the probe tip being suitable for delivery of light during medical applications, the probe tip comprising:
a cap portion and an elongated member extending outwardly from the cap portion, wherein:
i. the probe tip is designed to be removably fastened to the hand piece;
ii. the elongated member is configured to guide light from a proximate end to a distal end of the elongated member;
iii. the probe tip is a monolithic structure formed of a single optical material that includes plastic, polymer, epoxy or urethane;
iv. the elongated member is unclad.

2. A probe tip as in Claim 1 wherein the cap portion of the probe tip defines an opening for receiving a portion of the hand piece for removably fastening the tip to the hand piece.

3. A probe tip as in Claim 1 or Claim 2 wherein the elongated member is a taper.

4. A probe tip as in any of the preceding claims wherein the probe tip is configured to experience plastic deformation upon removal of the tip from the hand piece such that the tip is unsuitable for re-use.

5. A probe tip as in Claim 4 wherein the plastic deformation occurs at weakened areas of the tip that are thinned relative to other portions.

6. A probe tip as in any of the preceding claims wherein the material of the probe tip comprises PMMA.

7. A probe tip as in any of the preceding claims wherein the elongated member is arced and tapered from adjacent a proximate end to a distal end thereof.

## Patentansprüche

1. Sondenspitze zur Verwendung mit einem Handstück, welches mit einer Lichtquelle verbindbar ist, wobei die Sondenspitze dazu geeignet ist, während medizinischer Anwendungen Licht zu liefern, wobei die Sondenspitze aufweist:
einen Kappenabschnitt und ein langgestrecktes Element, das sich von dem Kappenabschnitt nach außen erstreckt, wobei:
i. die Sondenspitze dazu ausgelegt ist, abnehmbar an dem Handstück befestigt zu werden;
ii. das langgestreckte Element dazu eingerichtet ist, Licht von einem nahen Ende zu einem fernen Ende des langgestreckten Elementes zu leiten;
iii. die Sondenspitze eine monolithische Struktur ist, die aus einem einzigen optischen Material gebildet ist, welches Plastik, Polymer, Epoxid oder Urethan enthält;
iv. das langgestreckte Element unbekleidet ist.

2. Sondenspitze nach Anspruch 1, wobei der Kappenabschnitt der Probenspitze eine Öffnung definiert zum Empfangen eines Abschnitts des Handstücks, um die Spitze abnehmbar an dem Handstück zu befestigen.

3. Sondenspitze nach Anspruch 1 oder Anspruch 2, wobei das langgestreckte Element eine Verjüngung ist.

4. Sondenspitze nach einem der vorangehenden Ansprüche, wobei die Sondenspitze dazu eingerichtet ist, nach Abnehmen der Spitze von dem Handstück eine plastische Verformung zu erfahren, so dass die Spitze für eine Wiederverwendung ungeeignet ist.

5. Sondenspitze nach Anspruch 4, wobei die plastische Verformung an geschwächten Bereichen der Spitze auftritt, welche im Vergleich zu anderen Abschnitten dünner sind.

6. Sondenspitze nach einem der vorangehenden Ansprüche, wobei das Material der Sondenspitze PMMA aufweist.

7. Sondenspitze gemäß einem der vorangehenden Ansprüche, wobei das langgestreckte Element gewölbt ist und sich von einer Stelle neben einem nahen Ende zu einem fernen Ende des Elements verjüngt.

## Revendications

1. Pointe de sonde destinée à servir avec un embout à main qui peut se connecter à une source de lumière, la pointe de sonde permettant un apport de lumière pendant des applications médicales, la pointe de sonde comprenant :
un capot et un élément allongé s'étendant vers l'extérieur depuis le capot,
i. la pointe de sonde étant conçue pour être fixée d'une manière séparable à l'embout à main ;
ii. l'élément allongé étant agencé pour guider de la lumière depuis une extrémité proximale jusqu'à une extrémité distale de l'élément allongé ;
iii. la pointe de sonde étant une structure monolithique constituée d'une seule matière optique composée d'une matière plastique, d'un polymère, d'une résine époxy ou d'uréthane ;
iv. l'élément allongé n'est pas gainé.

2. Pointe de sonde selon la revendication 1, le capot de la pointe de sonde définissant une ouverture destinée à recevoir une partie de l'embout à main pour fixer d'une manière séparable la pointe à l'embout à main.

3. Pointe de sonde selon la revendication 1 ou la revendication 2, l'élément allongé étant un cône.

4. Pointe de sonde selon l'une quelconque des revendications précédentes, la pointe de sonde étant agencée pour subir, lorsqu'on retire la pointe de l'embout à main, une déformation plastique telle que la pointe n'est pas réutilisable.

5. Pointe de sonde selon la revendication 4, la déformation plastique se produisant dans des zones affaiblies de la pointe, qui sont amincies par rapport à d'autres parties.

6. Pointe de sonde selon l'une quelconque des revendications précédentes, la matière de la pointe de sonde étant constituée de PMMA.

7. Pointe de sonde selon l'une quelconque des revendications précédentes, l'élément allongé étant arqué et conique depuis les abords d'une extrémité proximale jusqu'à une extrémité distale de celui-ci.
